# EUROPEAN PATENT APPLICATION

(11) **EP 2 543 410 A1**
(43) Date of publication of application: **09.01.2013**
(21) Application number: 12001132.5
(22) Date of filing: 20.02.2012
(51) Int. Cl.: A61M 31/00

(54) **Suppository applicator**

(30) Priority: 07.07.2011 ES 201130738
(71) Applicant: Pedro Silvestre GEA, 08820 Prat de Llobregat (ES)
(72) Inventor: Pedro Silvestre GEA, 08820 Prat de Llobregat (ES)
(74) Representative: Durán Benejam, Luis

(57) **Abstract**

The suppository applicator comprises a hollow tubular body (1) having at the front end a rounded mouth (2) and at the rear end thereof fixed support wings (3), there being coupled to this second end a pushing element (4) that is movable longitudinally Inside tubular body (1). The applicator comprises a movable end stop (5) assembled around tubular body (1). Tubular body (1) has a graded scale (11) arranged longitudinally.

## Description

### Aim of the Invention

This invention relates to a suppository applicator, intended to facilitate the insertion of suppositories via the rectum.

### Field of application of the invention

This applicator is applicable in the sector of articles for administering medicines.

### Background to the Invention

Placing suppositories via the rectum is relatively simple and fast for a user who is suitably mobile; however, when the patient has mobility problems and requires the help of an assistant, a serious problem arises.

This happens, for example, in geriatric centres, hospitals or places attending the elderly, as the nurse or carer who is in charge of putting the suppository in place has certain hygienic problems to face, which are usually overcome by using gloves.

Even so, the feel and care that the actual user has, cannot be achieved by another person who is putting the suppository in place. In these situations the user or patient is uncomfortable with the handling, as haemorrhoids are rubbed and other discomforts occur, in addition to the delicate position with respect to the other person. Also, in elderly people or people who have problems controlling their sphincter muscles, as well as the hygiene problem for the person attending, there is also the question of whether or not the suppository has been correctly positioned or will be ejected by the patient's body,

Another drawback that arises is that when handling the suppository beforehand, it softens and becomes deformed due to the warmth, and this hinders applying it to the patient.

The applicant of this invention is unaware of the existence of any background that satisfactorily resolves the problem exposed.

### Description of the invention

The suppository applicator, which is the object of this invention, has a series of technical properties aimed at facilitating the insertion of suppositories via the rectum, irrespective of whether this operation is performed by the actual patient or a carer.

According to the invention, the suppository applicator comprises a hollow tubular body, having at the front end a rounded mouth and at the rear end fixed support wings, there being coupled to this rear end a pushing element that is movable longitudinally inside the tubular body.

Thus, the tubular body of the applicator is easy to Insert into the anus until it is housed deep enough inside the rectum, and then the suppository is placed in position via the action of the user on the pushing element, shifting the suppository previously held inside the tubular body, until it emerges from the front end. The rounded mouth of this front end of the tubular body prevents irritating the anus and tearing the rectum by rubbing it, for example against haemorrhoids, sensitive areas and the anal sphincter muscle. Also, it ensures that the suppository is inserted effectively, unlike the manual technique, where the suppository usually slips in the person's hands and becomes deformed by the heat. Subsequently removing the applicator is simple and clean, and said applicator can be disposed of if it is made from a low cost recyclable material.

In one embodiment it has been envisaged that the applicator comprises a movable end stop assembled around the tubular body. This movable end stop, for example extended transversally with respect to the tubular body, limits the part of the tubular body that can be inserted into the rectum. This control over the depth that the applicator is inserted into the rectum determines the position in which the suppository is placed. The movable end stop is particularly useful when placing suppositories in people who, owing to their delicate condition or who have problems with their sphincter muscles, require the suppository to be inserted deeply. Using this movable end stop in a position near the front end of the tubular body is also suitable when the opposite effect to that described above is required, In other words, when placing the suppository in a part of the rectum that is nearest the anus so as to avoid irritations, but preventing it from coming out.

In one embodiment, the tubular body has a graded scale arranged longitudinally on the surface thereof, which enables positioning the end stop accurately according to the dose instructions of the suppository to be administered.

### Description of the figures.

To complement this description and in order to facilitate understanding of the characteristics of the invention, a set of drawings is attached to this specification which, in a non-limiting manner, represent the following:

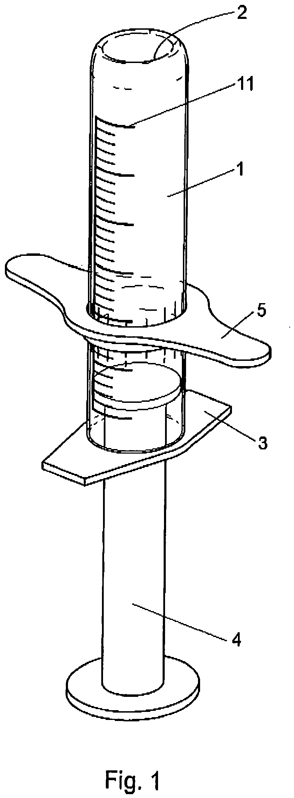

- Figure 1 shows a perspective view of the applicator with the pushing element in the less inserted position.

### Preferable embodiment of the invention

As can be seen in the referenced figure, the suppository applicator comprises a hollow tubular body (1) having at the front end a rounded mouth (2), intended to be inserted via the anus of the patient to position the suppository in a more or less deeper position in the rectum. At the rear end of tubular body (1) there are fixed wings (3), projecting on two sides, for supporting and correctly handling the applicator. At this rear end of tubular body (1) a pushing element (4) of the suppository is inserted, to cause said suppository to exit from the inside of tubular body (1) via front rounded mouth (2), once part of tubular body (1) has been inserted into the rectum.

Tubular body (1) has a movable end stop (5) assembled on the outer surface thereof, so that it is easy to position, but does not slide accidentally. This movable end stop (5) is ring shaped, and extends transversally with respect to tubular body (1), in two opposite projections. The positioning of these movable end stop (5) along tubular body (1) makes It possible to define the part of said tubular body (1) that can be inserted into the patient's rectum,

In one embodiment, tubular body (1) has a graded scale (11) arranged longitudinally on the surface thereof.

Having sufficiently described the nature of the invention, as well as a preferable embodiment, it is noted, for all effects and purposes, that the materials, shape, size and arrangement of the elements described can be modified, providing this does not imply altering the essential characteristics of the invention, which are claimed below,

## Claims

1. Suppository applicator, **characterised in that** it comprises a hollow tubular body (1) having at the front end thereof a rounded mouth (2) and at the rear end thereof fixed support wings (3), there being coupled to this second end a pushing element (4) that is movable longitudinally inside tubular body (1); and **in that** it comprises a movable end stop (5) assembled around tubular body (1).

2. Applicator according to claim 1, **characterised in that** tubular body (1) has a graded scale (11) arranged longitudinally.
